(19) **Europäisches Patentamt**
**European Patent Office**
**Office européen des brevets**

(11) **EP 2 695 172 B1**

(12) **EUROPÄISCHE PATENTSCHRIFT**

(45) Veröffentlichungstag und Bekanntmachung des Hinweises auf die Patenterteilung:
**22.02.2017 Patentblatt 2017/08**

(21) Anmeldenummer: **11712562.5**

(22) Anmeldetag: **06.04.2011**

(51) Int Cl.:
*G01R 33/12* (2006.01)          *H01F 7/20* (2006.01)
*A61B 5/05* (2006.01)

(86) Internationale Anmeldenummer:
**PCT/EP2011/055376**

(87) Internationale Veröffentlichungsnummer:
**WO 2012/136255 (11.10.2012 Gazette 2012/41)**

(54) **SPULENSYSTEM FÜR MAGNETIC PARTICLE IMAGING MIT MINIMIERTEN OHMSCHEN VERLUSTEN**

COIL SYSTEM FOR MAGNETIC PARTICLE IMAGING WITH MINIMIZED NON-REACTIVE LOSSES

SYSTÈME DE BOBINES POUR IMAGERIE PAR PARTICULES MAGNÉTIQUES AVEC RÉDUCTION DES PERTES OHMIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**12.02.2014 Patentblatt 2014/07**

(73) Patentinhaber: **Universität zu Lübeck**
**23538 Lübeck (DE)**

(72) Erfinder:
• **KNOPP, Tobias**
**23562 Lübeck (DE)**
• **SATTEL, Timo**
**22848 Norderstedt (DE)**
• **BUZUG, Torsten**
**23627 Groß Sarau (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A2- 1 286 173          WO-A2-2009/008956**
**JP-A- 2009 056 232**

• **KNOPP TOBIAS ET AL: "Generation of a static magnetic field-free line using two Maxwell coil pairs", APPLIED PHYSICS LETTERS, AIP, AMERICAN INSTITUTE OF PHYSICS, MELVILLE, NY, US, Bd. 97, Nr. 9, 1. September 2010 (2010-09-01), Seiten 92505-92505, XP012138986, ISSN: 0003-6951, DOI: 10.1063/1.3486118**

**Beschreibung**

Die Erfindung betrifft ein Spulensystem zur Erzeugung eines Magnetfeldes mit einem feldfreien Punkt, das beim "Magnetic Particle Imaging" einsetzbar ist.

Beim Magnetic Particle Imaging (MPI) werden lokale Konzentrationen magnetisierbarer Nanopartikel in einer räumlichen Verteilung, z.B. in einem lebenden Organismus, ermittelt.

Die Nanopartikel, beispielsweise Eisenoxidpartikel, werden durch ein externes, homogenes und mit vorgegebener Frequenz oszillierendes Magnetfeld (Aussteuerungsfeld, "drive field") zeitlich periodisch magnetisiert, wobei die Magnetisierung der Partikel in der Untersuchungsregion nicht-linear von der externen Feldstärke abhängt. Detektiert man das Zeitverhalten der Magnetisierung z.B. mit einer Empfangsspule, so weist das Signal höhere Harmonische der Frequenz des Aussteuerungsfeldes auf, und aus den zugehörigen Fourierkomponenten kann auf die Partikelkonzentration geschlossen werden.

Zur Eingrenzung kleiner Volumina der Untersuchungsregion werden starke Selektionsmagnetfelder mit den Aussteuerungsfeldern überlagert. Ein Selektionsmagnetfeld ist zeitlich konstant und weist an einem vorbestimmten Punkt der Untersuchungsregion eine Nullstelle auf. Ausgehend von diesem so genannten feldfreien Punkt (FFP) steigt das Selektionsmagnetfeld in alle Richtungen schnell an, so dass magnetisierbare Nanopartikel schon in geringem Abstand zum FFP in die magnetische Sättigung gelangen. Sie reagieren dann nicht mehr auf das zeitabhängige Aussteuerungsfeld und tragen entsprechend nicht mehr zum detektierten Signal bei. Dies bedeutet, das detektierbare Signal der Partikelmagnetisierung stammt allein aus der lokalen Umgebung des FFP und gibt über die dort vorhandene Partikelkonzentration Auskunft.

Das Selektionsfeld muss in der Nähe des FFP einen hohen Feldgradienten aufweisen. Unter dem Gradienten eines Magnetfeldes ist grundsätzlich der Vektorgradient bzw. die Jacobi-Matrix zu verstehen. Diese ist definiert als

$$\underline{\underline{J}} = (\underline{\nabla} \otimes \underline{H})^T = \begin{pmatrix} \dfrac{\partial H_x}{\partial x} & \dfrac{\partial H_x}{\partial y} & \dfrac{\partial H_x}{\partial z} \\[2mm] \dfrac{\partial H_y}{\partial x} & \dfrac{\partial H_y}{\partial y} & \dfrac{\partial H_y}{\partial z} \\[2mm] \dfrac{\partial H_z}{\partial x} & \dfrac{\partial H_z}{\partial y} & \dfrac{\partial H_z}{\partial z} \end{pmatrix}$$

mit dem Symbol $\otimes$ zur Kennzeichnung des dyadischen Produkts aus dem Nabla-Operator $\underline{\nabla}$ und dem Magnetfeld $\underline{H}$ sowie dem Superskript *T* als Notation der transponierten Matrix. In der Umgebung eines FFP ist $\underline{\underline{J}}$ eine Diagonalmatrix. Aus der Quellenfreiheit von Magnetfeldern folgt, dass ihre Spur Null ist. Wenn zudem aus Symmetriegründen zwei der Diagonalelemente denselben Wert annehmen, dann reicht es aus, ein Diagonalelement der Matrix zu bestimmen, um die gesamte Matrix zu erhalten. Im Folgenden soll deshalb der Wert der Komponente $\dfrac{\partial H_z}{\partial z}$ abkürzend als Magnetfeldgradient bezeichnet werden.

Zur Erzeugung eines solchen Feldes kann beispielsweise ein Paar gleichartiger, koaxialer und entlang der gemeinsamen Achse beabstandeter Spulen dienen, die gegenläufig von Strömen durchflossen sind. Die Untersuchungsregion befindet sich zwischen den Spulen. Sind die Ströme dem Betrage nach gleich, so befindet sich in der Mitte zwischen den Spulen auf der Symmetrieachse offenbar ein FFP. Der Feldgradient im FFP kann durch gleichartiges Vergrößern der Ströme erhöht werden, ohne dass die Lage des FFP geändert wird. Verändert man die Ströme in den Spulen nicht gleichartig, so verschiebt sich der FFP entlang der gemeinsamen Symmetrieachse.

Das Verschieben des FFP entlang der Symmetrieachse erlaubt das Durchführen eines Linienscans in der Untersuchungsregion. Es ist von Vorteil, den FFP ohne mechanische Bewegungen allein durch Steuern der Spulenströme positionieren zu können. Allerdings verhält es sich bei der Anordnung zweier Spulen mit dazwischen befindlicher Untersuchungsregion so, dass die erforderliche Stromstärke in jener Spule erheblich ansteigt, von der der FFP wegbewegt werden soll. Dies ist notwendig, um einen vorbestimmten Magnetfeldgradienten im FFP aufrecht zu erhalten.

Aus der Arbeit von Sattel et al., "Single-sided device for magnetic particle imaging", J. Phys. D: Applied Physics, 42(1): 1-5 (2009) ist eine koaxiale und koplanare Anordnung zweier Spulen zur Erzeugung eines magnetischen Selektionsfeldes für das MPI bekannt. Dabei werden eine innere (kleinerer Durchmesser) und eine äußere (größerer Durchmesser) Spule, die in einer gemeinsamen Ebene ineinander gesetzt angeordnet sind, gegensinnig von Strom durchflossen. Auch hier kann ein FFP mit großem Magnetfeldgradienten auf der gemeinsamen Symmetrieachse der Spulen erzeugt und durch Steuern der Ströme entlang dieser Achse - insbesondere auch außerhalb der Spulenebene - verschoben werden. Der Vorteil der dort beschriebenen Vorrichtung ist u. a. darin zu sehen, dass sich das Selektionsfeld mit einem frei beweglichen Applikator an ein zu untersuchendes Objekt heranführen lässt, etwa mit der Hand oder mit einem Roboterarm. Insbesondere Messungen an einem lebenden Patienten können dadurch sehr vereinfacht werden.

Die Strecke, um die der FFP bei vorgegebenem Magnetfeldgradienten aus der Spulenebene heraus und in die Untersuchungsregion hinein bewegt werden kann, ist jedoch auch bei der einseitigen Anordnung durch die dafür erforderlichen Spulenströme begrenzt. Diese steigen in beiden Spulen mit dem Abstand des FFP zur Spulenebene stark an. Die koaxiale, koplanare Spulenanordnung nach Sattel et al. soll nachfolgend auch als konzentrisch bezeichnet werden.

Es ist ferner bekannt, Spulenanordnungen, die zur Erzeugung magnetischer Gradientenfelder ausgebildet sind, durch wenigstens eine weiter außen liegende Spule zu erweitern, die zur Abschirmung des Magnetfeldes dient. Insbesondere der US 6,765,381 B2 ist eine Anordnung aus zwei entlang einer gemeinsamen Symmetrieachse beabstandeten, gleichartigen konzentrischen Spulen zu entnehmen, in deren Innen- und Außenspulen jeweils paarweise gleichgroße, entgegen gesetzte Ströme fließen sollen, um ein möglichst lineares magnetisches Gradientenfeld zu erzeugen. Die äußeren Spulen sind dabei als Abschirmspulen ("shield coils") gedacht. Ein FFP wird bei dieser Anordnung in der Mitte zwischen den Spulen gebildet. Die Veränderung seiner Position ist nicht vorgesehen.

Des weiteren offenbart WO2009/008956 A2 ein Spulensystem zur Erzeugung eines Magnetfeldes gemäß dem Oberbegriff des Anspruchs 1.

Die Aufgabe der Erfindung besteht darin, ein Spulensystem vorzuschlagen, das ein magnetisches Gradientenfeld mit einem FFP an einem wählbaren Ort auf einer vorbestimmten Strecke und mit einem vorbestimmten Feldgradienten im FFP erzeugt, wobei der Gesamtstrombedarf und somit die ohmschen Verluste minimal sind.

Die Aufgabe wird gelöst durch ein Spulensystem zur Erzeugung eines Magnetfeldes mit einem feldfreien Punkt an einer vorgebbaren Position auf einer vorgegebenen Strecke und mit einem vorgebbaren Magnetfeldgradienten im feldfreien Punkt gekennzeichnet durch N ≥ 2 koplanare Spulen angeordnet am ersten Ende der Strecke und M ≥ 2 koplanare Spulen am zweiten Ende der Strecke, wobei alle Spulen koaxial ausgerichtet sind, und eine Steuereinrichtung, die angepasst ist, um die Ströme in den N + M Spulen so zu steuern, dass bei jeder Vorgabe wenigstens der Position des feldfreien Punktes auf der Strecke und des Magnetfeldgradienten im feldfreien Punkt jene Spulenströme, die zu minimalen ohmschen Verlusten führen, aus der Lösung eines linearen Gleichungssystems bestimmt und eingestellt werden. Die Unteransprüche geben vorteilhafte Ausgestaltungen des Spulensystems an.

Das erfindungsgemäße Spulensystem umfasst eine Spulenanordnung und eine Steuereinrichtung. Die Spulenanordnung wird dabei aus zwei koaxialen und entlang der Symmetrieachse beabstandeten, konzentrischen Spulen gebildet.

Unter einer konzentrischen Spule im Sinne der Erfindung ist eine Anordnung von N in derselben Ebene ineinander gesetzten (i. F. koplanaren) Spulen $L_1$, ..., $L_N$ mit Radien $R_1 < ... < R_N$ zu verstehen, die jeweils einzeln von Strömen $I_1$, ..., IN nach Vorgabe durch die Steuereinrichtung durchflossen werden können. Dabei ist N ≥ 2 eine natürliche Zahl.

Ohne Beschränkung der Allgemeinheit sei eine erste konzentrische Spule in der Ebene z = 0 und eine zweite in der Ebene $z = z_0 > 0$ angeordnet. Die gemeinsame Symmetrieachse der Spulen steht senkrecht auf beide Ebenen. Der feldfreie Punkt (FFP) liegt auf dieser Achse bei der Koordinate $z = z_P \in [0, z_0]$, die im Übrigen frei wählbar ist.

Die zweite konzentrische Spule bei $z = z_0$ kann identisch zur ersten Spule bei z = 0 aufgebaut sein. Sie kann aber auch aus M Spulen $L_1$, ..., $L_M$ mit Radien $R_1 < ... < R_M$ bestehen, wobei N ≠ M ≥ 2 gelten soll.

Die Ströme $I_1$, ..., IN der ersten Spule und $I_1$, ..., IM der zweiten sind voneinander unabhängige Variablen und können als Komponenten des K-dimensionalen Spaltenvektors (K = N + M)

$$(1) \qquad \underline{I} = \begin{pmatrix} I_1 \\ \vdots \\ I_K \end{pmatrix}$$

geschrieben werden. Als Konvention sollen die ersten N Komponenten die Ströme der ersten konzentrischen Spule beschreiben und die Komponenten N+1 bis K die der zweiten Spule. Die Vorzeichen der Ströme sollen die Fließrichtung angeben.

Die Steuereinrichtung des erfindungsgemäßen Spulensystems ist nun dazu ausgebildet, dass sie bei Vorgabe der Koordinate $z_P$, an der ein FFP mit einem definierten Feldgradienten erzeugt werden soll, einen Vektor gemäß Gleichung (1) bestimmt und die Spulenströme gemäß den Komponenten dieses Vektors einstellt.

Befinden sich die konzentrischen Spulen in einer festen Anordnung, insbesondere einem festen Abstand $z_0$, und soll der FFP mit einer festen Schrittweite und einem festen Magnetfeldgradienten über eine vorbekannte Stützstellenfolge verschoben werden, dann genügt es bereits, eine Tabelle mit vorab berechneten Strömen im Speicher der Steuereinrichtung vorzuhalten. Die Steuereinrichtung weist somit wenigstens einen elektronischen Datenspeicher auf, in dem eine Tabelle mit Spulenströmen für eine Auswahl von Vorgaben wenigstens der Position des feldfreien Punktes auf der Strecke und des Magnetfeldgradienten im feldfreien Punkt hinterlegt ist, die zuvor durch Lösen eines linearen Gleichungssystems bestimmt worden sind.

Die tabellierten Ströme können nach dem weiter unten dargestellten Verfahren bestimmt werden, um minimale ohmsche Verluste zu garantieren.

Besonders bevorzugt wird aber die Steuereinrichtung so ausgebildet, dass sie die Berechnungen der Ströme selbst durchführen kann, wenn etwa der Spulenabstand $z_0$ verändert oder die Koordinate des FFP und/oder der Magnetfeldgradient im FFP durch direkte Eingabe des Nutzers vorgegeben wird. Eine erfindungsgemäße Steuereinrichtung kann insbesondere ein herkömmlicher Computer mit einer ansteuerbaren Schnittstelle zur Regelung von Starkströmen sein.

Der nachfolgend dargestellte Algorithmus dient der Berechnung eines Vektors gemäß Gleichung (1) bei Vorgabe von $z_P$, $z_0$ und Magnetfeldgradient G im FFP, der auf das gewünschte Magnetfeld führt und zugleich die geringsten ohmschen Verluste verursacht. Im Anschluss werden Simulationsergebnisse diskutiert, die die Energieeinsparung verdeutlichen. Dabei zeigt:

Fig. 1    Simulationsergebnisse für zwei identische konzentrische Spulen (N = M) im Vergleich zu einer herkömmlichen Spulenanordnung nach dem Stand der Technik (gestrichelte Linie);

Fig. 2    Simulierte Magnetfeldstärken entlang der Strecke [0, $z_0$] für verschiedene Positionen $z_P$ des FFP. Gezeigt sind Feldverläufe der herkömmlichen Spulenanordnung nach dem Stand der Technik (gestrichelte Linie) und der in Anspruch 1 beschriebenen Spulenanordnung für N = M = 2.

Das am Ort $z \in [0, z_0]$ durch die erfindungsgemäße Spulenanordnung erzeugte Magnetfeld ergibt sich durch Superposition der Felder der Spulen $L_1, ..., L_K$ nach dem Biot-Savart-Gesetz (Komponentenkonvention wie bei Gleichung (1)). Es weist nur eine z-Komponente im FFP auf. Das Magnetfeld jeder Spule $L_j$ ist an einem gegebenen Ort z der Stromstärke $I_j$ (j = 1, ..., K) proportional. Die Proportionalitätskonstante wird als Spulenempfindlichkeit ("coil sensitivity") bezeichnet und sei hier als $S_j(z)$ notiert.

$$(2) \qquad H_z(z) = \sum_{j=1}^{K} S_j(z) I_j$$

Die Spulenempfindlichkeiten tragen die Ortsabhängigkeit des Magnetfeldes und können als bekannt für alle z vorausgesetzt werden. Sie sind für jede der konzentrischen Spulen nur einmal zu bestimmen und liegen vorzugsweise tabelliert in der Steuereinrichtung vor. Falls erforderlich, können sie auf Stützstellen, für die keine Einträge vorliegen, interpoliert werden.

Wie bereits erläutert, genügt die Angabe eines Diagonalelementes der Jacobi-Matrix, um den Vektorgradienten des Magnetfeldes $\underline{H}$ in der Umgebung des FFP festzulegen, hier

$$(3) \qquad \frac{\partial H_z}{\partial z}(z) = \sum_{j=1}^{K} \frac{\partial S_j(z)}{\partial z} I_j \qquad .$$

Definiert man die 2xK-Matrix

$$(4) \qquad \underline{\underline{S}}(z_P) = \begin{pmatrix} S_1 & \cdots & S_K \\ \partial S_1/\partial z & & \partial S_K/\partial z \end{pmatrix} \Bigg|_{z=z_P} \qquad ,$$

dann ist die Aufgabe, einen FFP mit Magnetfeldgradient $\dfrac{\partial H_z}{\partial z} = G$ am Ort z = $z_P$ bereitzustellen, äquivalent mit der Lösung des linearen Gleichungssystems

$$(5) \qquad \underline{G} = \begin{pmatrix} 0 \\ G \end{pmatrix} = \underline{\underline{S}}\,\underline{I} \qquad ,$$

wobei $\underline{G}$ ein zweidimensionaler Spaltenvektor, $\underline{I}$ der Stromvektor aus Gleichung (1) und das Produkt aus der Matrix $\underline{\underline{S}}$ und $\underline{I}$ im Sinne der gewöhnlichen Matrixmultiplikation auszuführen ist.

Das Gleichungssystem (5) ist unterbestimmt und besitzt unendlich viele Lösungen. Da jede Differenz zweier Lösungen auch die homogene Gleichung

$$(6) \qquad \underline{0} = \begin{pmatrix} 0 \\ 0 \end{pmatrix} = \underline{\underline{S}}\,\underline{I}_0$$

löst, ist die allgemeine Lösungsmannigfaltigkeit von (5) die Linearkombination aus irgendeiner Lösung von (5) und der Lösungsmannigfaltigkeit von (6). Letztere ist der Kern (oder Nullraum) der 2×K-Matrix $\underline{\underline{S}}$, die für jede Vorgabe von $z_P$ gemäß Gleichung (4) aufzustellen ist. Der Nullraum ist ein Untervektorraum jenes K-dimensionalen Vektorraumes, der alle $\underline{I}$ enthält. Er ist offenbar disjunkt zur Lösungsmannigfaltigkeit von Gleichung (5), d.h. je eine Lösung von Gleichung (6) und eine von Gleichung (5) besitzen einen von Null verschiedenen Abstand zueinander.

Betrachtet man die gewichtete Norm

$$(7) \qquad P = \underline{I}^T \underline{\underline{R}}\,\underline{I} = \sum_{i,j=1}^{K} I_i R_{ij} I_j = \sum_{j=1}^{K} R_j I_j^2$$

mit $I^T$ als transponiertem Stromvektor und diagonaler Gewichtsmatrix $\underline{\underline{R}}$ mit $R_{ij} = \delta_{ij} R_j \geq 0$ für alle j = 1, ..., K ($\delta_{ij}$ - Kroneckersymbol) für irgendeine Lösung $\underline{I}$ des Gleichungssystems (5), dann stellt man fest, dass die Norm minimal wird, wenn die Beziehungen

$$(8) \qquad \underline{I}^T \underline{\underline{R}}\,\underline{I}_0 = 0 \quad \text{oder auch} \quad \underline{I}_0^T \underline{\underline{R}}\,\underline{I} = 0$$

für alle Vektoren $\underline{I}_0$ erfüllt sind, die das Gleichungssystem (6) lösen. Denn setzt man (8) voraus und berechnet nun die Norm einer anderen Lösung von Gleichung (5), so muss diese Lösung durch $\hat{\underline{I}} = \underline{I} + \underline{I}_0$ mit irgendeinem $\underline{I}_0$ beschrieben werden können, und man erhält

$$(9) \qquad P = \hat{\underline{I}}^T \underline{\underline{R}}\,\hat{\underline{I}} = (\underline{I} + \underline{I}_0)^T \underline{\underline{R}}(\underline{I} + \underline{I}_0) = \underline{I}^T \underline{\underline{R}}\,\underline{I} + \underbrace{\underline{I}^T \underline{\underline{R}}\,\underline{I}_0 + \underline{I}_0^T \underline{\underline{R}}\,\underline{I}}_{=0} + \underbrace{\underline{I}_0^T \underline{\underline{R}}\,\underline{I}_0}_{>0}$$

Die gewichtete Norm wird also nur größer für Lösungen des Gleichungssystems (5), die nicht mehr die Bedingung (8) erfüllen.

Es ist zweckmäßig, die gewichtete Norm nach Gleichung (7) als ohmsche Verlustleistung der erfindungsgemäßen Spulenanordnung aufzufassen, indem die Komponenten $R_j$ mit den Ohmschen Widerständen der Spulen $L_j$ für alle j=1, ..., K identifiziert werden.

Die Gewichtsmatrix $\underline{\underline{R}}$ ist offenbar trivial invertierbar, denn $\underline{\underline{R}}^{-1}$ ist ebenfalls diagonal und enthält die Leitwerte der Spulenanordnung. Aus Gleichung (6) folgt aber nun

$$(10) \qquad \underline{\underline{S}}\,\underline{I}_0 = \underline{\underline{S}}\,\underline{\underline{R}}^{-1} \underline{\underline{R}}\,\underline{I}_0 = \underline{0} \qquad \text{oder auch} \qquad \underline{I}_0^T \underline{\underline{S}}^T = \underline{I}_0^T \underline{\underline{R}}\,\underline{\underline{R}}^{-1} \underline{\underline{S}}^T = \underline{0}$$

und man sieht sofort, dass jeder beliebige zweidimensionale Spaltenvektor $\underline{y}$ über

$$(11) \qquad \widetilde{\underline{I}} = \underline{\underline{R}}^{-1} \underline{\underline{S}}^T \underline{y} \qquad \text{bzw.} \qquad \widetilde{\underline{I}}^T = \underline{y}^T \underline{\underline{S}}\,\underline{\underline{R}}^{-1}$$

auf einen K-dimensionalen Stromvektor führt, der der Bedingung (8) genügt. Setzt man schließlich Gleichung (11) in Gleichung (5) ein, ergibt sich ein eindeutig lösbares Gleichungssystem (zwei Gleichungen mit zwei Unbekannten).

$$(12) \quad \underline{G} = \underline{\underline{S}}\, \underline{\underline{R}}^{-1}\, \underline{\underline{S}}^{T}\, \underline{y}$$

Um also mit der erfindungsgemäßen Spulenanordnung zweier konzentrischer Spulen (N koplanare Spulen bei $z = 0$, M koplanare Spulen bei $z = z_0$, N, M $\geq$ 2, z-Achse als gemeinsame Symmetrieachse) einen feldfreien Punkt und einen Magnetfeldgradienten G ($=\partial H/\partial z$) bei der Koordinate $z_P$ einzurichten, hat die Steuereinrichtung folgendes zu bestimmen:

Die Matrix $\underline{S}$ gemäß Gleichung (4) ist aus vorbekannten, tabellierten Daten der Spulenempfindlichkeit für den gewählten Punkt $z_P$ aufzustellen. Dabei wird auch der Spulenabstand $z_0$ berücksichtigt; insbesondere kann auch seine Veränderung in das Aufstellen der Matrix eingehen.

Die Ohmsche Matrix $\underline{R}$ ist vorab bekannt und i. d. R. konstant.

Das Gleichungssystem (12) ist zu lösen, und die Lösung ist eindeutig.

Einsetzen der Lösung von (12) in (11) ergibt eben jene Lösung $\underline{I}$ von Gleichungssystem (5) mit der minimalen Norm (7).

Die Steuereinrichtung steuert dann die Ströme in den einzelnen Spulen der Spulenanordnung gemäß der berechneten Komponenten der Lösung $\underline{I}$. Das Spulensystem umfassend Spulenanordnung und Steuereinrichtung erzeugt das gewünschte Magnetfeld somit automatisch mit minimalen Ohmschen Verlusten bzw. geringstem Stromverbrauch.

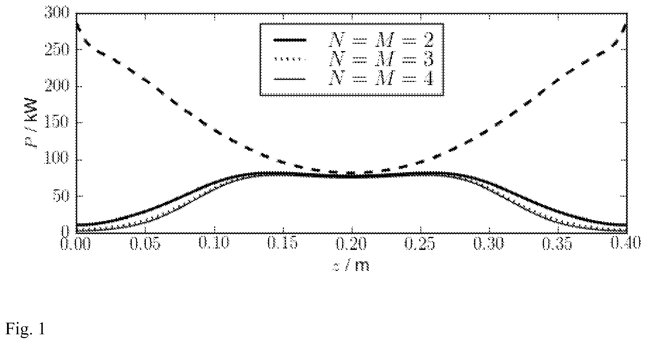

In Fig. 1 ist eine Simulationsrechnung zu sehen, die die Leistungsersparnis verdeutlicht. Die gestrichtelte Kurve gibt den Leistungsbedarf in Kilowatt für ein Spulensystem nach dem Stand der Technik (zwei koaxiale, gleiche Spulen bei $z = 0$ und bei $z = z_0 = 40$ cm, gegensinnig von Strömen durchflossen) wieder. Die übrigen Linien (s. Inlay) beschreiben die Leistungsaufnahme für zwei konzentrische Spulen mit N = M = 2, 3, 4 bei Steuerung der Ströme nach dem oben beschriebenen Algorithmus. Wie man sieht, hat das erfindungsgemäße Spulensystem keinen nennenswerten Vorteil gegenüber dem Stand der Technik, wenn der FFP genau in der Mitte zwischen den Spulenebenen eingerichtet sein soll. Bei jeder anderen Position des FFP auf der Symmetrieachse werden die Vorteile aber sehr deutlich, insbesondere an den Rändern des Messintervalls, d.h. in unmittelbarer Nähe einer der beiden konzentrischen Spulen.

Bereits bei N = M = 2 ist die Leistungsersparnis signifikant im gesamten zentralen Messintervall. Größere N und/oder M bringen zusätzliche, aber anteilig geringere, Verbesserungen an den Messintervallgrenzen.

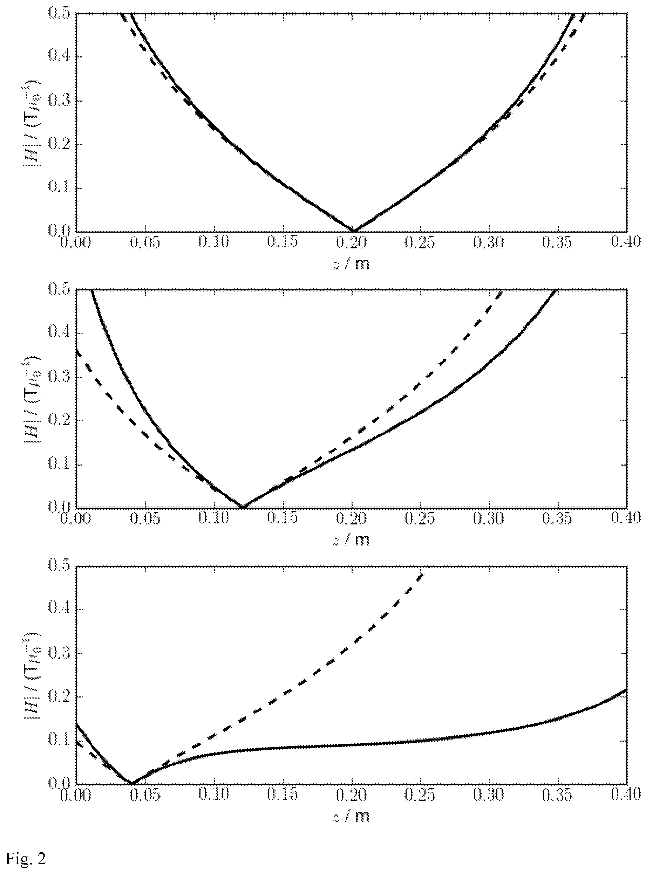

Fig. 2 zeigt die Magnetfeldstärken für das Spulensystem mit N = M = 2 (durchgezogen) und für Spulen nach dem Stand der Technik, wenn der FFP zu verschiedenen Positionen auf der z-Achse verschoben wird. Insgesamt ist das Magnetfeld mit dem erfindungsgemäßen Spulensystem schwächer als nach dem Stand der Technik, wenn der FFP in der Nähe einer der konzentrischen Spulen positioniert wird. Die geringere magnetische Feldenergie ist eine Folge der Optimalität der von der Steuereinrichtung eingestellten Ströme. Der Feldgradient im FFP ist in allen Konfigurationen gleich.

Die konzentrische Spulenanordnung mit N = M = 2 ist bereits der US 6,765,381 B2 zu entnehmen. Sie ist jedoch für sich genommen nicht in der Lage, die gestellte Aufgabe zu lösen. Hierfür ist ferner eine Steuereinrichtung erforderlich, die die Spulenströme der jeweils gewünschten Lage des FFP und des Magnetfeldgradienten G anpasst. Zudem kann die Steuereinrichtung einen ggf. veränderlichen Spulenabstand berücksichtigen. Konzentrische Spulenanordnungen mit N > 2 oder M > 2 wurden für die Erfindungsaufgabe noch nicht vorgeschlagen.

Die Steuereinrichtung kann besonders einfach ausgebildet sein, wenn alle Messparameter (z.B. FFP-Position, Gradient G, Spulenabstand $z_0$) vorab festgelegt und die Berechnungen der Ströme wie oben beschrieben bereits durchgeführt worden sind. In diesem Fall kann die Steuereinrichtung die Ströme durch Ablesen einer gespeicherten Tabelle steuern. Ebenso kann die Steuereinrichtung einen Prozessor aufweisen, der zur Interpolation der im elektronischen Datenspeicher hinterlegten Daten angepasst ist, um die Ströme in Situationen zu steuern, in denen Zwischenwerte - etwa der FFP-Position - abgefragt werden, die nicht in den Tabellen enthalten sind.

Besonders bevorzugt führt die Steuereinrichtung jedoch die hier beschriebenen Berechnungen bei jeder Vorgabe neuer Messparameter einfach neu aus. Die Berechnungen stellen auf heutigen Mikroprozessoren keinerlei Herausforderung dar.

**Patentansprüche**

1. Spulensystem zur Erzeugung eines Magnetfeldes mit einem feldfreien Punkt an einer vorgebbaren Position auf einer vorgegebenen Strecke und mit einem vorgebbaren Magnetfeldgradienten im feldfreien Punkt mit N $\geq$ 2 koplanaren Spulen angeordnet am ersten Ende der Strecke und M $\geq$ 2 koplanaren Spulen am zweiten Ende der Strecke, wobei alle Spulen koaxial ausgerichtet sind, und mit einer Steuereinrichtung, **dadurch gekennzeichnet, dass** die Steuereinrichtung angepasst ist, um Ströme in den N + M Spulen so zu steuern, dass bei jeder Vorgabe wenigstens der Position des feldfreien Punktes auf der Strecke und des Magnetfeldgradienten im feldfreien Punkt jene Spulenströme, die zu minimalen ohmschen Verlusten führen, aus der Lösung eines linearen Gleichungssystems bestimmt und eingestellt werden, so dass an der vorgegebenen Position der feldfreie Punkt erzeugt wird und so dass der vorgegebene Magnetfeldgradient erzeugt wird.

2. Spulensystem nach Anspruch 1, **dadurch gekennzeichnet, dass** die Steuereinrichtung wenigstens einen elektronischen Datenspeicher aufweist, in dem eine Tabelle mit Spulenströmen für eine Auswahl von Vorgaben wenigstens der Position des feldfreien Punktes auf der Strecke und des Magnetfeldgradienten im feldfreien Punkt hinterlegt ist, die zuvor durch Lösen eines linearen Gleichungssystems bestimmt worden sind.

3. Spulensystem nach Anspruch 2, **dadurch gekennzeichnet, dass** die Steuereinrichtung einen Prozessor aufweist, der zur Interpolation der im elektronischen Datenspeicher hinterlegten Daten angepasst ist.

4. Spulensystem nach Anspruch 1, **dadurch gekennzeichnet, dass** die Steuereinrichtung einen elektronischen Datenspeicher und einen Mikroprozessor aufweist, wobei der Datenspeicher tabellierte Daten für die ohmschen Widerstände und für die Spulenempfindlichkeiten der N + M Spulen wenigstens für eine Auswahl von Punkten auf der Strecke enthält und der Mikroprozessor angepasst ist, um bei Vorgabe wenigstens der Position des feldfreien Punktes $z_P$ auf der Strecke und des Magnetfeldgradienten G im feldfreien Punkt die folgenden Schritte auszuführen:

a. Aufstellen einer 2$\times$(N+M) Matrix $\underline{\underline{S}}(z_P) = \left( \begin{array}{ccc} S_1 & \cdots & S_{N+M} \\ \partial S_1/\partial z & \cdots & \partial S_{N+M}/\partial z \end{array} \right)\Bigg|_{z=z_P}$ aus den tabellierten Daten der Spulenempfindlichkeiten für den gewählten Punkt $z_P$

b. Aufstellen einer (N+M)x(N+M) Diagonalmatrix $\underline{R}$ aus den tabellierten ohmschen Widerständen

c. Lösen des linearen Gleichungssystems $\left( \begin{array}{c} 0 \\ G \end{array} \right) = \underline{\underline{S}}\,\underline{R}^{-1}\,\underline{\underline{S}}^T\,\underline{y}$

d. Berechnen des (N+M)-dimensionalen Stromvektors $\underline{I}=\underline{R}^{-1}\underline{\underline{S}}^T\underline{y}$, dessen Komponenten als Ströme in den N+M Spulen eingerichtet werden.

**Claims**

1. Coil system for generating a magnetic field having a field-free point at a predeterminable position on a predetermined line and having a predeterminable magnetic field gradient at the field-free point, comprising N $\geq$ 2 coplanar coils disposed at the first end of the line and M $\geq$ 2 coplanar coils at the second end of the line, wherein all coils are coaxially oriented, and a control device, **characterized in that** the control device is configured to control the currents in the N + M coils in such a manner that for each predetermination of at least the position of the field-free point on the line and of the magnetic field gradient at the field-free point those coil currents which result in minimal ohmic losses are determined from the solution of a linear system of equations and are set, such that at the predetermined position the field-free point is generated and the predetermined magnetic field gradient is generated.

2. Coil system according to claim 1, **characterized in that** the control device comprises at least one electronic data memory in which a table including coil currents for a selection of predeterminations of at least the position of the field-free point on the line and of the magnetic field gradient at the field-free point is stored which have been predetermined by solving a linear system of equations.

3. Coil system according to claim 2, **characterized in that** the control device comprises a processor which is arranged for interpolating the data stored in the electronic data memory.

**4.** Coil system according to claim 1, **characterized in that** the control device comprises an electronic data memory and a microprocessor, wherein the data memory contains tabulated data for the ohmic resistances and for the coil sensitivities of the N + M coils for at least a selection of points on the line, and that the microprocessor is configured to, upon predetermination of at least the position of the field-free point $Z_P$ on the line and of the magnetic field gradient G at the field-free point, carry out the following steps:

a. determining a 2x(N+M) matrix $\underline{\underline{S}}(z_P) = \left. \begin{pmatrix} S_1 & \cdots & S_{N+M} \\ \partial S_1/\partial z & & \partial S_{N+M}/\partial z \end{pmatrix} \right|_{z=z_P}$ from the tabulated data of the coil sensitivities for the selected point $Z_P$

b. determining a (N+M)x(N+M) diagonal matrix $\underline{R}$ from the tabulated ohmic resistances

c. solving the linear system of equations $\begin{pmatrix} 0 \\ G \end{pmatrix} = \underline{\underline{S}}\,\underline{R}^{-1}\,\underline{\underline{S}}^{T}\,\underline{y}$

d. calculating the (N+M)-dimensional current vector $\underline{I} = \underline{R}^{-1}\underline{\underline{S}}^{T}\underline{y}$, the components of which are set as currents in the N+M coils.

## Revendications

**1.** Système de bobines destiné à la génération d'un champ magnétique présentant un point à champ nul à une position pouvant être prédéfinie, sur un trajet prédéfini, et présentant un gradient de champ magnétique pouvant être prédéfini, au point à champ nul, avec N ≥ 2 bobines coplanaires disposées à la première extrémité du trajet et M ≥ 2 bobines coplanaires à la deuxième extrémité du trajet, toutes les bobines étant orientées coaxialement, et comprenant un dispositif de commande, **caractérisé en ce que** le dispositif de commande est adapté pour commander les courants dans les N + M bobines de telle sorte que lors de chaque prédéfinition au moins de la position du point à champ nul sur le trajet et du gradient de champ magnétique au point à champ nul, ceux des courants de bobine qui engendrent des pertes ohmiques minimes soient déterminés par résolution d'un système d'équations linéaires et soient réglés, de sorte que le point à champ nul est généré à la position prédéfinie et de sorte que le gradient de champ magnétique prédéfini est produit.

**2.** Système de bobines selon la revendication 1, **caractérisé en ce que** le dispositif de commande présente au moins une mémoire de données électronique dans laquelle est enregistré un tableau avec des courants de bobine pour une sélection de prédéfinitions au moins de la position du point à champ nul sur le trajet et du gradient de champ magnétique au point à champ nul, tableau qui a été déterminé préalablement par résolution d'un système d'équations linéaires.

**3.** Système de bobines selon la revendication 2, **caractérisé en ce que** le dispositif de commande présente un processeur qui est adapté en vue de l'interpolation des données enregistrées dans la mémoire de données électronique.

**4.** Système de bobines selon la revendication 1, **caractérisé en ce que** le dispositif de commande présente une mémoire de données électronique et un microprocesseur, la mémoire de données contenant des données présentées sous forme de tableau pour les résistances ohmiques et pour les sensibilités de bobine des N + M bobines, au moins pour une sélection de points sur le trajet, et le microprocesseur est adapté pour exécuter les étapes suivantes, lors de la prédéfinition au moins de la position du point à champ nul $z_p$ sur le trajet et du gradient de champ magnétique G au point à champ nul :

a. établissement d'une matrice 2x(N+M)

$$\underline{\underline{S}}(z_P) = \left. \begin{pmatrix} S_1 & \cdots & S_{N+M} \\ \partial S_1/\partial z & & \partial S_{N+M}/\partial z \end{pmatrix} \right|_{z=z_P}$$

à partir des données des sensibilités de bobines présentées sous forme de tableau pour le point $z_p$ sélectionné ;
b. établissement d'une matrice diagonale (N+M)x(N+M) $\underline{R}$ à partir des résistances ohmiques présentées sous forme de tableau ;

c. résolution du système d'équations linéaires $\begin{pmatrix} 0 \\ G \end{pmatrix} = \underline{\underline{S}}\,\underline{R}^{-1}\,\underline{\underline{S}}^{T}\,\underline{y}$

d. calcul du vecteur de courant $\underline{I}=\underline{R}^{-1}\underline{\underline{S}}^{T}\underline{y}$ de dimension (N+M), dont les composantes sont installées en tant que courants dans les N+M bobines.

$\begin{pmatrix} 0 \\ G \end{pmatrix} = \underline{\underline{S}}\,\underline{R}^{-1}\,\underline{\underline{S}}^{T}\,\underline{y}$

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6765381 B2

- WO 2009008956 A2

**In der Beschreibung aufgeführte Nicht-Patentliteratur**

- **SATTEL et al.** Single-sided device for magnetic particle imaging. *J. Phys. D: Applied Physics,* 2009, vol. 42 (1), 1-5